# DEMANDE DE BREVET EUROPEEN

(11) **EP 2 149 600 A1**
(43) Date de publication de la demande: **03.02.2010**
(21) Numéro de dépôt: 09176125.4
(22) Date de dépôt: 28.02.2005
(51) Int. Cl.: C12N 1/02, C12M 1/12, A23L 1/30

(54) **Concentrât liquide de bactéries adaptées et viables pour un usage alimentaire**

(30) Priorité: 27.02.2004 FR 0401997
(62) Demande divisionnaire de: 05732472.5
(71) Demandeur: COMPAGNIE GERVAIS DANONE, 75009 Paris (FR)
(72) Inventeur: La désignation de l'inventeur n'a pas encore été déposée
(74) Mandataire: Warcoin, Jacques

(57) **Abrégé**

La présente invention concerne un concentrât liquide de bactéries lactiques adaptées et viables pour un usage alimentaire.

## Description

La présente invention concerne un concentrât liquide de bactéries lactiques adaptées et viables pour un usage alimentaire.

L'ingestion de certaines souches de bactéries, en particulier celles qui appartiennent aux genres *Lactobacillus* et *Bifidobacterium* sont particulièrement bénéfiques au niveau de la santé, notamment en favorisant un bon fonctionnement de la flore intestinale. En effet, ces bactéries produisent des bactériocines et de l'acide lactique qui augmentent la digestibilité des aliments, favorisent le péristaltisme intestinal, et accélèrent l'évacuation des selles. De plus, ces bactéries produisent certaines vitamines du complexe B, et favorisent en général l'absorption des vitamines et minéraux, diminuent le cholestérol sanguin, renforcent le système immunitaire et tapissent les muqueuses intestinales afin de protéger contre l'invasion et les activités des microorganismes nuisibles.

De ce fait, depuis plusieurs années, les industries agroalimentaires tentent d'incorporer de telles bactéries dans leurs produits finaux, le plus généralement des yaourts.

Actuellement, ces bactéries sont utilisées sous une forme congelée ou lyophilisée. Cependant, ces processus de production sont traumatisants pour les bactéries qui perdent une partie de leur activité et parfois leur viabilité. Cela est préjudiciable pour les industriels producteurs et pour les consommateurs de ces produits car les bactéries doivent satisfaire aux exigences de qualité et de performances technologiques, si possible pendant plusieurs mois. Il serait donc souhaitable d'utiliser des bactéries produites par un procédé leur assurant une viabilité et une activité maximale. A cet effet, une méthode consiste à produire les bactéries sous une forme liquide. Cependant il a été mis en évidence que cette méthode génère également une mortalité importante des bactéries, après l'introduction des bactéries dans le produit final.

En outre, pour diminuer les coûts de stockage des bactéries et faciliter l'adjonction des bactéries dans le produit final, il serait souhaitable de concentrer les bactéries sous forme liquide. Pour cela, l'homme du métier utilise de manière habituelle une étape de centrifugation ou de filtration. Cependant, la centrifugation est un processus traumatisant pour les bactéries qui peut entraîner une forte mortalité cellulaire notamment due à un fort cisaillement et de plus, ce procédé n'est pas bien adapté pour la centrifugation de faibles volumes tels que ceux requis dans la production de bactéries destinées à être additionnées en tant que probiotique à des produits alimentaires. Concernant une étape de filtration classique, celle-ci pose également des problèmes mortalité des bactéries et de colmatage des filtres par les bactéries.

Il serait donc souhaitable de produire un volume souhaité de concentrât liquide de bactéries qui présentent une activité et une viabilité maximale après l'étape de concentration et après l'introduction dans le produit final.

De manière surprenante et inattendue, les inventeurs ont montré qu'une étape d'adaptation des bactéries permettait d'augmenter de manière significative l'activité et la viabilité des bactéries après l'introduction dans le produit final.

De plus, les inventeurs ont montré qu'une étape de filtration tangentielle, sous certaines conditions particulières (pression, concentration, porosité de membrane etc), permettait de concentrer de gros volumes de culture de bactéries, tout en préservant leur viabilité et sans colmatage des filtres.

La filtration tangentielle permet de produire deux courants en fonction de la nature et de la structure de la membrane : le perméat (le milieu de culture sensiblement exempt de bactéries) et le retentât (contenant les bactéries, appelé aussi concentrât). Dans une filtration tangentielle, le fluide ne circule non pas perpendiculairement mais parallèlement à la surface de la membrane et assure ainsi par sa vitesse d'écoulement un auto nettoyage qui prévient l'accumulation de dépôts qui obturent la surface de filtration (appelé communément colmatage des filtres).

Un objet de la présente invention est donc un concentrât de bactéries **caractérisé en ce que** le concentrât est liquide et en ce que les bactéries sont adaptées, viables et à une concentration comprise entre 5.10¹⁰ et 5.10¹¹ ufc/ml.

Par bactéries, on entend désigner selon la présente invention des bactéries lactiques, du genre *Lactobacillus spp., Bifidobacterium spp., Streptococcus spp, Lactococcus spp.* et en particulier *Lactobacillus casei, Lactobacillus plantarum, Lactobacillus bulgaricus, Lactobacillus helveticus, Lactobacillus acidophilus, Bifidobacterium animalis, Bifidobacterium breve, Streptococcus thermophilus, Lactococcus lactis.*

Par bactéries adaptées, on entend désigner, selon la présente invention, des bactéries plus résistantes à différents stress, en particulier liés à différents stress physicochimiques.

Par bactéries adaptées et viables, on entend désigner, selon la présente invention, des bactéries dont le taux de survie après 28 jours dans un produit alimentaire, en particulier un produit laitier ou une boisson, est supérieur à 60% et avantageusement supérieur à 80%.

La viabilité des bactéries est mesurée par des techniques de numération connue de l'homme du métier comme par exemple la numération en masse, la numération en surface, les cellules de Malassez, le comptage direct, la turbidité, la néphélométrie, le comptage électronique, la cytométrie en flux, la fluorescence, l'impédimétrie, l'analyse d'images.

Selon la présente invention, le concentrât est **caractérisé en ce que** les bactéries adaptées présentent au moins l'une des caractéristiques suivantes quand elles sont ajoutées à un produit alimentaire :
i) un taux de survie supérieur à 80% après 14 jours dans un produit alimentaire à une température comprise entre 4°C et 45°C, ledit produit alimentaire ayant un pH compris entre 3 et 7 ou
ii) un taux de survie supérieur à 60% et avantageusement supérieur à 80% après 28 jours dans un produit alimentaire à une température comprise entre 4°C et 45°C, ledit produit alimentaire ayant un pH compris entre 3 et 7.

Selon la présente invention, le concentrât est **caractérisé en ce que** les bactéries présentent les deux caractéristiques i) et ii).

Selon la présente invention, le concentrât est **caractérisé en ce que** le produit alimentaire est un produit laitier et/ou une boisson.

Selon la présente invention, les bactéries du concentrât sont viables pendant une période comprise entre 4 et 6 semaines.

Selon la présente invention, le concentrât est **caractérisé en ce qu**'il est susceptible d'être obtenu par le procédé comprenant les étapes successives de propagation des bactéries dans un milieu de culture, d'adaptation des bactéries, de lavage du milieu de culture contenant les bactéries adaptées par microfiltration tangentielle, et de concentration en bactéries du milieu lavé par microfiltration tangentielle.

Selon la présente invention, le milieu de culture de l'étape de propagation est un milieu synthétique.

Par milieu synthétique, on entend désigner selon la présente invention un milieu dans lequel sont introduits des composants soumis à un contrôle quantitatif et qualitatif rigoureux.

Selon la présente invention, la solution utilisée à l'étape de lavage est adaptée à l'utilisation alimentaire du concentrât de bactéries et présente une pression climatique compatible avec la viabilité des bactéries.

Les inventeurs ont montré que l'étape d'adaptation des bactéries permet de réduire la mortalité des celles-ci, provoquée par le changement de milieu des bactéries entre leur milieu de culture et le produit alimentaire final à additiver.

Selon la présente invention l'adaptation des bactéries est mise en évidence par la mesure de paramètres du milieu de culture des bactéries et/ou de paramètres des bactéries. Selon la présente invention, les paramètres du milieu de culture sont préférentiellement le pH, la pression osmotique et/ou la température.

D'autres paramètres du milieu de culture des bactéries pour la mise en évidence de l'adaptation des bactéries sont possibles, comme par exemple la concentration en sucre du milieu bactérien.

Préférentiellement, dans le cas où le paramètre du milieu de culture est le pH, l'étape d'adaptation est réalisée par diminution du pH par acidification naturelle.

Afin d'effectuer l'étape d'adaptation des bactéries au pH par acidification naturelle, on peut par exemple mesurer la concentration en sucre du milieu de fermentation et au-delà d'une concentration seuil pour chaque espèce de bactéries, on sait que le pH n'est plus régulé et l'adaptation au milieu devient très aisée.

Ainsi, par exemple, si la concentration en sucre du milieu de fermentation de Lactobacillus casei est de 9 g/L, le pH n'est plus régulé et est environ égal à 5. Il devient alors plus aisé pour la souche adaptée d'être ajoutée à un nouveau milieu et ceci permet une viabilité plus importante des bactéries dans le milieu final.

En outre, selon la présente invention, la filtration tangentielle peut être utilisée pour l'étape d'adaptation des bactéries.

Selon la présente invention la ou les membranes de filtration tangentielle sont d'une porosité comprise entre 0.01 et 0,5µm et de manière préférentielle, entre 0.1 et 0.3 µm.

Ces membranes sont utilisées pour les étapes de lavage et de concentration du procédé et éventuellement l'étape d'adaptation des bactéries.

Les membranes de filtration sont caractérisées par:
- la porosité et l'épaisseur de la couche filtrante dont dépend le débit de perméat.
- le diamètre des pores et leur répartition dont dépend l'efficacité de séparation.
- le matériau employé dont dépend la résistance mécanique, chimique, thermique et la facilité de nettoyage.

Par membrane de filtration, on entend désigner des membranes organiques ou minérales.

Les membranes organiques peuvent être composées entre autre d'acétate de cellulose, de polyamides aromatiques, de polysulfone, d'esters de cellulose, de cellulose, de nitrate de cellulose, de PVC, ou de Polypropylène.

Les membranes minérales peuvent être composées entre autre de céramique frittée, de métal fritté, de carbone, ou de verre.

Selon la présente invention, le paramètre des bactéries est la taille des bactéries.

Préférentiellement, dans le cas où l'adaptation est mise en évidence par la taille des bactéries, la distribution des longueurs de chaque bactérie dudit concentrât se situe majoritairement entre 0,1 et 10 micromètres, avantageusement entre 0,5 et 5 micromètres.
La mesure de la taille des bactéries se fait par un moyen adapté.
Un moyen adapté peut être par exemple un prélèvement régulier de bactéries suivi d'une mesure de la taille des bactéries par cytométrie de flux.

Selon la présente invention, le pH du concentrât est compris entre 3 et 6.

Selon la présente invention, la température d'utilisation du concentrât est comprise entre 25 et 45°C et préférentiellement entre 35 et 39°C.

Par température d'utilisation, on entend désigner selon la présente invention la température du concentrât quand il est ajouté à un produit alimentaire.

Selon la présente invention, le concentrât est conditionné dans des poches souples, hermétiques et stériles.

Par poches souples hermétiques, on entend désigner, selon la présente invention et de manière préférentielle, des poches en plastique alimentaire.

Selon la présente invention, le concentrât, conditionné en poches souples hermétiques, peut être conservé à une température comprise entre -50°C et 4°C après conditionnement.

De manière optionnelle, il est possible de rajouter au concentrât liquide de bactéries adaptées et viables conditionné en poches souples, et conservé à des températures basses, des molécules cryoprotectrices telles que le saccharose par exemple.

Selon la présente invention, le concentrât, conditionné en poches souples et hermétiques, conservé à une température comprise entre -50°C et 4°C, est réchauffé jusqu'à une température comprise entre 25°C et 45°C, avantageusement entre 35 et 39°C par un moyen adapté avant d'être utilisé.

Par moyen adapté, on entend désigner selon la présente invention par exemple l'utilisation d'un bain marie à une température non létale pour les bactéries, par exemple 37°C.

Un objet de la présente invention est également l'utilisation du concentrat liquide de bactéries adaptées et viables, selon la présente invention en tant qu'additif alimentaire.

Par additif alimentaire, on entend désigner selon la présente invention toute substance chimique ajoutée aux aliments durant leur préparation ou en vue de leur entreposage pour obtenir un effet technique désiré. De plus, selon la présente invention, le concentrât liquide de bactéries possède une numération stable, les bactéries étant viables et n'effectuant pas de fermentation dans le produit final additivé.

Un objet de la présente invention est également un produit alimentaire additivé, **caractérisé en ce que** l'additif alimentaire utilisé est le concentrât liquide de bactéries adaptées et viables selon la présente invention.

Selon la présente invention, le produit alimentaire est un produit laitier et/ou une boisson.

Par produit laitier on entend désigner selon la présente invention, en plus du lait, les produits dérivés du lait, tels la crème, la crème glacée, le beurre, le fromage, le yogourt; les produits secondaires, comme le lactosérum, la caséine ainsi que divers aliments préparés contenant comme ingrédient principal du lait ou des constituants du lait.

Par boisson on entend désigner selon la présente invention des boissons comme par exemple les jus de fruits, les mélanges de lait et de jus de fruits, les jus végétaux tels que par exemple le jus de soja, le jus d'avoine ou le jus de riz, les boissons alcoolisées comme par exemple le kéfir, les sodas, et les eaux des source ou minérales additionnées ou non de sucre ou d'arômes par exemple.

Un objet de la présente invention est également un procédé de fabrication d'un produit alimentaire additivé selon la présente invention, **caractérisé en ce que** le concentrât liquide de bactéries adaptées et viables est additionné au produit alimentaire en fin de ligne de production et préférentiellement avant le conditionnement du produit alimentaire.

Selon la présente invention, le procédé de fabrication d'un produit alimentaire additivé est **caractérisé en ce que** le concentrât liquide de bactéries adaptées et viables est additionné au produit alimentaire en ligne par pompage.

La présente invention sera mieux comprise à l'aide du complément de description qui va suivre, qui se réfère à des exemples de mesure de la viabilité et de l'adaptation des bactéries du concentrât liquide selon la présente invention.

Il va de soi, toutefois que ces exemples ne sont donnés qu'à titre d'illustration de l'objet de l'invention, dont ils ne sauraient constituer en aucune manière une limitation.

### Légende des figures:

- Figure 1 :: Suivi de la viabilité de souches de lactobacillus casei adaptées et non adaptées dans un produit alimentaire de type yaourt sur une période de 28 jours.
- Figure 2a :: Histogramme de distribution de taille de souches de Lactobacillus casei adaptées et non adaptées avant d'être soumises à un stress acide.
- Figure 2b :: Histogramme de distribution de taille de souches de Lactobacillus casei adaptées et non adaptées après être soumises à un stress acide.
- Figure 3 :: Courbes montrant l'adaptation de *Lactobacillus casei* par rapport à la température du milieu de culture (37°C et 39°C). La permittivité relative ε _{R} (quantité adimensionnelle égale à la permittivité ε, exprimée en pF/cm, divisée par la permittivité du vide ε₀) est exprimée en fonction de l'âge des bactéries (en heures).
- Figure 4 :: Courbes montrant l'adaptation de *Lactobacillus casei* en fonction de la pression osmotique (concentration en glucose de 20, 40 et 80 g/L, respectivement en gris clair, gris foncé et noir). La permittivité ε, exprimée en pF/cm, est exprimée en fonction de la Densité Optique (DO).

### EXEMPLES :

### * Exemple 1 : Conséquences de l'adaptation de souches de L. casei sur leur viabilité.

On souhaite évaluer les conséquences d'une étape d'adaptation de souches de Lactobacillus casei au niveau de leur viabilité.

Pour cela, on prépare un lot de bactéries témoin de Lactobacillus casei qui sont mises en culture dans un milieu MRS (milieu spécial permettant la croissance des lactobacilles, mis au point par Man, de Rogosa et Sharpe).

En parallèle à ce lot témoin, on prépare un lot de bactéries Lactobacillus casei, qui après la mise en culture dans un milieu MRS sont adaptées par une étape d'acidification naturelle.

Pour cela, après 17 heures de culture, une diminution du pH est réalisée par acidification naturelle sur une heure pour passer de pH 6,5 à pH 5.

Ensuite, les deux lots de bactéries sont lavés et concentrés en bactéries par microfiltration tangentielle.
Ces deux concentrât de bactéries sont introduit séparément dans une masse de yaourt à pH 5.5 et à une température de 10°C.

On mesure la quantité de bactéries vivantes à J+ 1 dans les deux lots de yaourt.

On prélève ensuite tous les jours un échantillon des deux lots de yaourt additionné respectivement du concentrât bactérien témoin et du concentrât de bactéries adaptées, et on quantifie le nombre de souches survivantes par rapport au nombre de souches vivantes à J+1. Pour cela, on effectue une numération en masse.

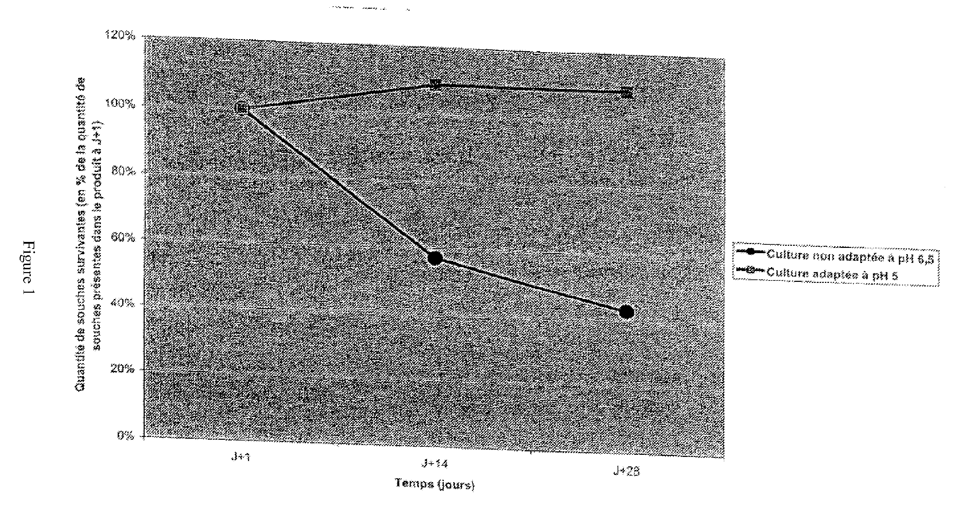

Pour chacune des mesures de viabilité durant la période de conservation du produit fini, ce dernier est bien homogénéisé avant le prélèvement. Un prélèvement stérile de 1 ml de produit est réalisé. Une dilution sériée de 10 en 10 est effectuée. Les différentes dilutions du produit sont placées dans une boite de Petri et un milieu gélosé liquide (puisque préalablement chauffé à 50°C) est coulé sur ces fractions du produit. On choisira le milieu à couler en fonction de la nature des bactéries que l'on souhaite compter. Le milieu gélosé durcit. Les boites de Petri sont alors placées en incubation pendant quelques jours (2-5j) à 37°C. Les résultats sont illustrés par la figure 1.

On observe qu'à au bout de 7 jours, le nombre de bactéries survivantes dans le lot des bactéries témoin est de 80% et pour le lot de bactéries adaptées de 105% (il y a eu une légère croissance bactérienne).

Au bout de 14 jours, le nombre de bactéries survivantes dans le lot des bactéries témoin est de 58% et pour le lot de bactéries adaptées de 110% (il y a eu une légère croissance bactérienne).

Au bout de 28 jours, le nombre de bactéries survivantes dans le lot des bactéries témoin est de 42% et pour le lot de bactéries adaptées de 110% (il y a eu une légère croissance bactérienne).

En conclusion, l'étape d'adaptation des bactéries induit une diminution de la mortalité des bactéries de l'ordre de 60 % par rapport à un lot témoin de bactéries non adaptées, après 28 jours dans un yaourt.

### * Exemple 2 : Evolution de la taille de bactéries adaptées et de bactéries non adaptées soumises à un stress acide.

On souhaite suivre l'évolution de la taille de bactéries adaptées et de bactéries non adaptées soumises à un stress acide.

Pour cela, on prépare un lot de bactéries témoin de Lactobacillus casei qui sont mises en culture dans un milieu MRS (milieu spécial permettant la croissance des lactobacilles, mis au point par Man, de Rogosa et Sharpe).

En parallèle à ce lot témoin, on prépare un lot de bactéries Lactobacillus casei, qui après la mise en culture dans un milieu MRS sont adaptées par une étape d'acidification naturelle.

Pour cela, après 17 heures de culture, une descente du pH est réalisée par acidification naturelle sur une heure pour passer de pH 6,5 à pH 5.

Ensuite, les deux lots de bactéries sont lavés et concentrés en bactéries par microfiltration tangentielle.

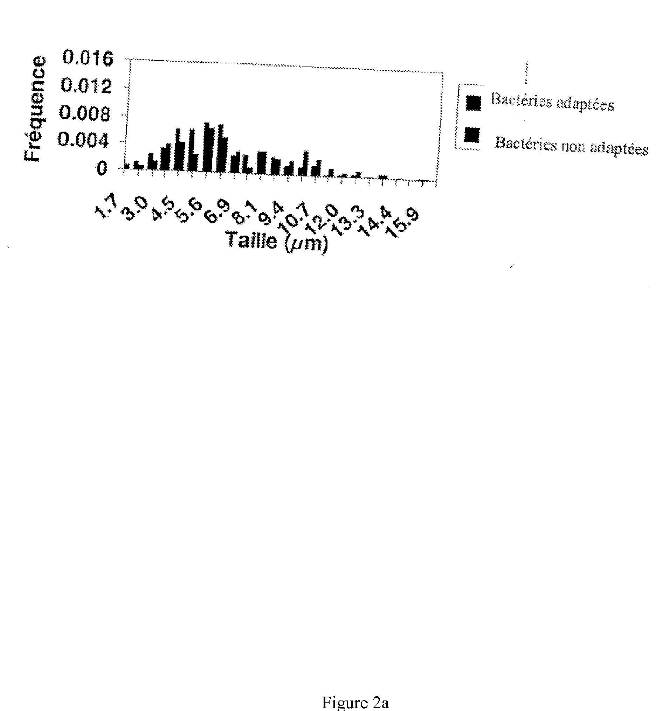

On effectue ensuite un prélèvement de bactéries et l'on mesure leur taille par cytométrie de flux. On établit ainsi un histogramme de distribution de taille des bactéries adaptées et non adaptées (lot témoin) (figure 2a). On observe que la distribution de taille des bactéries des deux lots est très similaire.

Ensuite, les deux lots de bactéries sont soumis à un stress acide par ajout des bactéries à un milieu ayant un pH de 3.

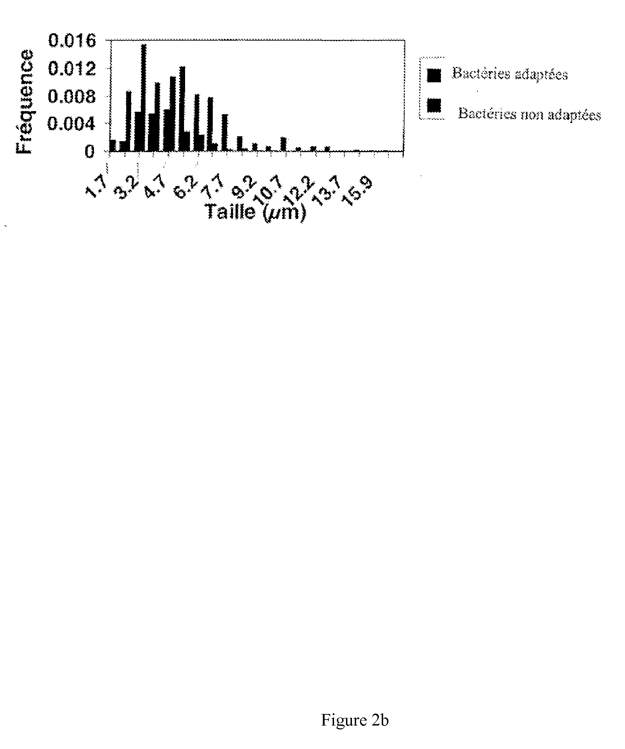

On effectue ensuite un prélèvement de bactéries et l'on mesure leur taille par cytométrie de flux. On établit ainsi un histogramme de distribution de taille des bactéries adaptées et non adaptées après un stress acide (figure 2b). On observe que la distribution de taille des bactéries des deux lots est très différente. Dans le lot de bactéries adaptées, la taille de fréquence la plus importante est de 3,2 µm (fréquence de 0.016). Dans le lot de bactéries non adaptées, la taille de fréquence la plus importante est de 5.45 µm (fréquence de 0.012).

En conclusion, l'étape d'adaptation des bactéries induit une diminution de la taille des bactéries de l'ordre de 60% lorsque celles-ci sont soumises à un stress acide, par rapport à des bactéries non adaptées. Il est donc possible de mettre en évidence l'adaptation des bactéries par la mesure de leur taille.

### * Exemple 3 : Mise en évidence de l'adaptation des bactéries par la mesure de l'influence du paramètre « température » du milieu de culture.

On souhaite mettre en évidence l'adaptation des bactéries en mesurant l'influence du paramètre du milieu de culture qu'est la température.

Pour cela, on prépare deux lots de bactéries de *Lactobacillus casei* à partir d'un même inoculum. Ces deux lots sont mis en culture dans deux milieux MRS (milieu spécial permettant la croissance des Lactobacilles, mis au point par Man, de Rogosa et Sharpe).

Une sonde de biomasse permettant de mesurer la permittivité relative ε_{R} est utilisée. Les sondes utilisables à cette fin sont connues de l'homme du métier (voir notamment FR2835921). La permittivité relative ε_{R} est une quantité adimensionnelle égale à la permittivité ε (exprimée en pF/cm) divisée par la permittivité du vide ε₀ (ε₀ = 8,854187.10⁻² pF/cm). L'évolution de la permittivité relative est mesurée au cours du temps. La permittivité relative sera fonction du nombre de cellules vivantes et de la taille de ces cellules.

Les deux milieux de culture rigoureusement identiques et comportant la même quantité de bactéries sont cultivés l'un à 37°C et l'autre à 39°C.

La quantité de souches augmente au cours du temps, ce qui est normal.

Les inventeurs ont pu vérifier que le nombre de cellules total n'était pas différent dans les deux milieux de culture. Une technique classique de mesure de densité optique (du type spectromètre d'absorbance) peut être utilisée à cette fin ou bien une sonde optique Wedgewood (système mesurant dans le proche infrarouge la densité optique de suspensions microbiennes). L'absorbance du milieu mesurée par un spectromètre sera fonction de la quantité de cellules totale dans le milieu. Des techniques de dénombrement sur boite peuvent être utilisées.

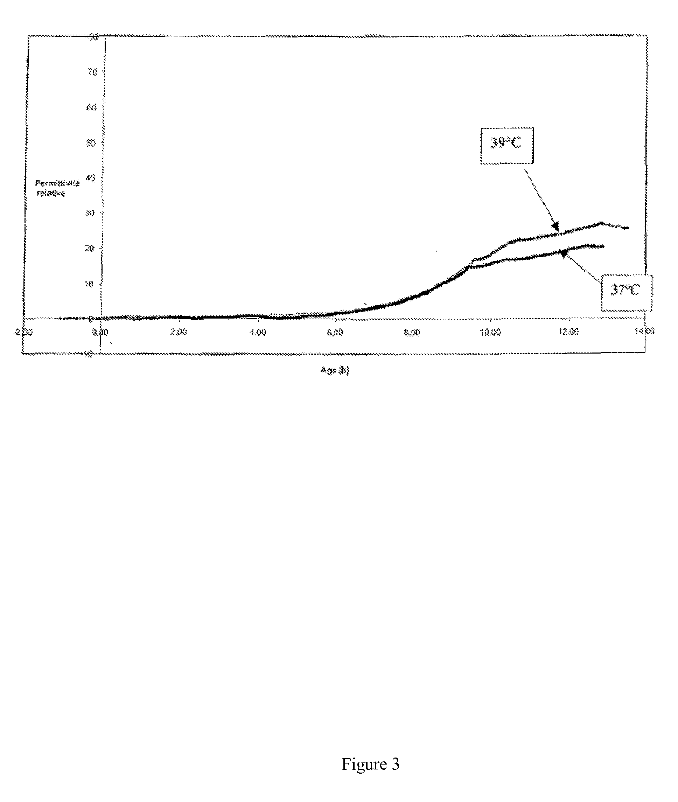

Grâce à ka sonde de biomasse utilisée, il a pu être mis en évidence que les bactéries changeaient de forme et de taille, donc s'adaptaient en fonction de la température du milieu de culture. La Figure 3 illustre cette observation.

### * Exemple 4 : Mise en évidence de l'adaptation des bactéries par la mesure de l'influence des paramètres « température » et « pH » du milieu de culture.

On souhaite mettre en évidence l'adaptation des bactéries en mesurant l'influence de deux paramètres du milieu de culture pris conjointement que sont la température et le pH.

Pour cela, on prépare trois lots de bactéries de *Lactobacillus casei* à partir d'un même inoculum.

On cultivera les trois milieux de culture à trois températures différentes (35, 37 et 39°C) tout en soumettant à chaque fois les bactéries à un stress acide en abaissant le pH du milieu à un pH de 3.

Le changement de taille des cellules traduira leur adaptation aux conditions du milieu. Cette taille sera mesurée par microscopie.

Dans le tableau 1, les résultats obtenus montrent bien que les bactéries s'adaptent en fonction des paramètres du milieu que sont la température et le pH puisque ces bactéries changent de taille.

**Tableau 1 : Influence de la température et du pH sur la taille des bactéries (exprimés en µm)**

| **Température (°C)** | **Taille moyenne des bactéries pendant la préculture** | **Taille moyenne des bactéries avant changement de pH** | **Taille moyenne des bactéries après changement de pH** |
|---|---|---|---|
| 35 | 3 | 3 | 2 |
| 37 | 4 | 5 | 4 |
| 39 | 3 | 7 | 5 |

### * Exemple 5 : Mise en évidence de l'adaptation des bactéries par la mesure de l'influence du paramètre « pression osmotique » du milieu de culture.

On souhaite mettre en évidence l'adaptation des bactéries en mesurant l'influence du paramètre du milieu de culture qu'est la pression osmotique.

Pour cela, on prépare trois lots de bactéries de *Lactobacillus casei* à partir d'un même inoculum. Ces lots sont mis en culture dans un milieu MRS (milieu spécial permettant la croissance des *Lactobacillus,* mis au point par Man, de Rogosa et Sharpe).

Les trois milieux de culture comportant la même quantité de bactéries contiendront respectivement des quantités de 20, 40 et 80 g de glucose par litre de milieu de culture. Plus la concentration en glucose du milieu est élevée, plus la pression osmotique de ce milieu est élevée.

Une sonde permettant de mesurer la permittivité relative est utilisée. Les sondes utilisables à cette fin sont connues de l'homme du métier (FR2835921). L'évolution de cette permittivité relative au cours du temps est mesurée. La permittivité ε (exprimée en pF/cm) est calculée en multipliant la permittivité relative ε_{R} mesurée par la permittivité du vide ε₀ (ε₀ = 8,854187.10⁻² pF/cm).

Une technique classique de mesure de densité optique (du type spectromètre d'absorbance), ou bien une sonde optique Wedgewood (système mesurant dans la proche infrarouge la densité optique de suspensions microbiennes), est utilisée pour mesurer l'évolution de la densité optique du milieu au cours du temps. La valeur de Densité Optique (DO) obtenue sera fonction du nombre de cellules total dans le milieu.

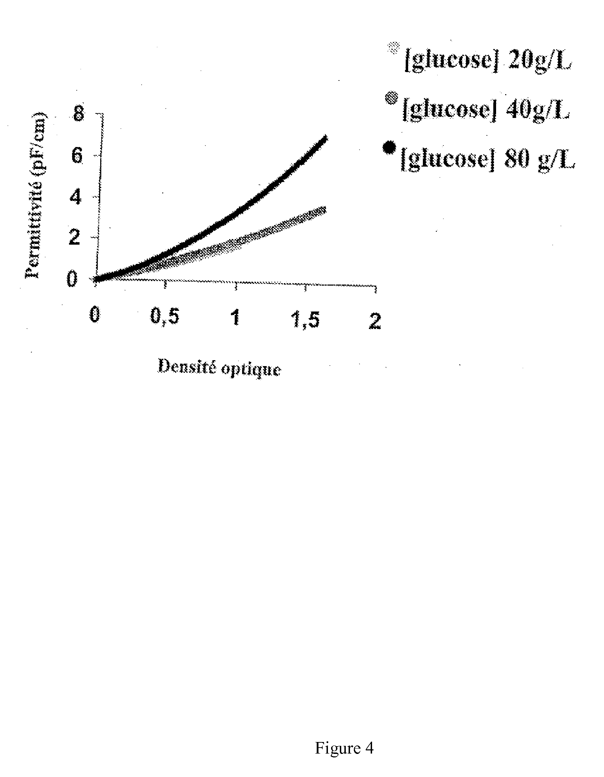

En exprimant la permittivité en fonction de la DO, la courbe résultante (Figure 4) permet de mettre en évidence l'évolution de la viabilité (exprimée par la mesure de la permittivité) et de la taille des cellules en fonction de la pression osmotique du milieu. Les résultats montrent que les bactéries changent de taille. En effet, dans le cas où il n'y aurait pas ce changement de taille des cellules, les résultats observés sur la Figure 4 seraient des droites rectilignes. Dans le cas présent, on peut observer des courbes (corrélation non linéaire).
Les bactéries s'adaptent donc en fonction de la pression osmotique du milieu de culture.

## Revendications

1. Concentrat liquide de bactéries lactiques, en particulier des bactéries du genre *Lactobacillus spp., Bifidobacterium spp. Streptococcus spp.* et *Lactococcus spp,* **caractérisé en ce que** les bactéries sont adaptées, viables, à une concentration comprise entre 5.10¹⁰ et 5.10¹¹ ufc/ml, lesdites bactéries adaptées étant plus résistantes à différents stress, en particulier liés à différents stress physicochimiques, et **en ce que** l'adaptation des bactéries est mise en évidence par la mesure de paramètres des bactéries et/ou de paramètres du milieu de culture des bactéries, lesdits paramètres du milieu de culture étant le pH, la pression osmotique, et/ou la température.

2. Concentrat selon la revendication 1 **caractérisé en ce que** le paramètre du milieu de culture est le pH et **en ce que** l'étape d'adaptation est réalisée par diminution du pH par acidification naturelle.

3. Concentrat selon la revendication 1 ou 2, **caractérisé en ce que** le paramètre des bactéries est la taille des bactéries.

4. Concentrat selon la revendication 3, **caractérisé en ce que** la distribution des longueurs de chaque bactérie dudit concentrat se situe majoritairement entre 0,1 et 10 micromètres, avantageusement entre 0,5 et 5 micromètres.

5. Concentrat selon l'une quelconque des revendications précédentes, **caractérisé en ce que** les bactéries adaptées présentent au moins l'une des caractéristiques suivantes quand elles sont ajoutées a un produit alimentaire :
i) un taux de survie supérieur à 80% après 14 jours dans un produit alimentaire à une température comprise entre 4°C et 45°C, ledit produit alimentaire ayant un pH compris entre 3 et 7 ou
ii) un taux de survie supérieur à 60% et avantageusement supérieur à 80% après 28 jours dans un produit alimentaire à une température comprise entre 4°C et 45°C, ledit produit alimentaire ayant un pH compris entre 3 et 7.

6. Concentrat selon la revendication 5, **caractérisé en ce que** les bactéries présentent les deux caractéristiques i) et ii).

7. Concentrat selon la revendication 5 ou 6, **caractérisé en ce que** le produit alimentaire est un produit laitier et/ou une boisson.

8. Concentrat selon l'une quelconque des revendications précédentes, **caractérisé en ce que** les bactéries sont viables pendant une période comprise entre 4 et 6 semaines.

9. Concentrat selon l'une quelconque des revendications précédentes, **caractérisé en ce que** son pH est compris entre 3 et 6.

10. Concentrat selon l'une quelconque des revendications 1 à 10, **caractérisé en ce qu'**il est conservé à une température comprise entre -50°C et +4°C après conditionnement.

11. Concentrat selon la revendication 10, **caractérisé en ce qu'**il est réchauffé jusqu'à une température comprise entre 25 et 45°C, avantageusement entre 35 et 39°C, par un moyen adapté avant d'être utilisé.

12. Utilisation du concentrat selon l'une quelconque des revendications 1 à 11 en tant qu'additif alimentaire.

13. Utilisation du concentrat selon l'une quelconque des revendications 1 à 11, à une température comprise entre 25 et 45°C, avantageusement entre 35 et 39°C.

14. Récipient sous la forme de poches souples, hermétiques et stériles comprenant le concentrat selon l'une quelconque des revendications 1 à 11.

15. Produit alimentaire additivé, **caractérisé en ce que** l'additif alimentaire utilisé est un concentrat liquide de bactéries adaptées et viables selon l'une quelconque des revendications 1 à 11.

16. Produit alimentaire additivé selon la revendication 15, **caractérisé en ce qu'**il s'agit d'un produit laitier et/ou d'une boisson.

17. Procédé de fabrication d'un produit alimentaire additivé selon la revendication 15 ou 16, **caractérisé en ce que** le concentrat liquide de bactéries adaptées et viables est additionné au produit alimentaire en fin de ligne de production et avantageusement avant le conditionnement du produit alimentaire.

18. Procédé de fabrication d'un produit alimentaire additivé selon la revendication 17, **caractérisé en ce que** le concentrat liquide de bactéries adaptées et viables est additionné au produit alimentaire en ligne par pompage.
